(19) Europäisches Patentamt
European Patent Office
Office européen des brevets

(11) Veröffentlichungsnummer : **0 112 588**
**B1**

(12) # EUROPÄISCHE PATENTSCHRIFT

(45) Veröffentlichungstag der Patentschrift : **11.11.87**

(21) Anmeldenummer : **83113175.0**

(22) Anmeldetag : **28.12.83**

(51) Int. Cl.⁴ : **C 07 C 39/12**, C 07 C 37/20, C 07 C 39/367, A 61 K 31/05, C 07 C 37/055, A 61 K 31/055

(54) **4-Hydroxy-alpha-äthyl-benzhydrole, Verfahren zu ihrer Herstellung und diese Verbindungen enthaltende Arzneimittel.**

(30) Priorität : **28.12.82 HU 418882**

(43) Veröffentlichungstag der Anmeldung : **04.07.84 Patentblatt 84/27**

(45) Bekanntmachung des Hinweises auf die Patenterteilung : **11.11.87 Patentblatt 87/46**

(84) Benannte Vertragsstaaten : **AT BE CH DE FR GB IT LI LU NL SE**

(56) Entgegenhaltungen :
**DE-A- 2 260 138**
**CHEMICAL ABSTRACTS, Band 96, Nr. 5, 1. Februar 1982, Seite 16, Nr. 28204a, Columbus, Ohio, US; SZINAI I. et al.: "Metabolism of 4-(2'-diethylamino)ethoxy-alpha-ethylbenzhydrol (RGH 6201) in rat urine and bile"**

(73) Patentinhaber : **RICHTER GEDEON VEGYESZETI GYAR R.T.**
**Gyömröi ut 19-21**
**H-1475 Budapest X (HU)**

(72) Erfinder : **Tòth, Edit, Dipl.-Ing.**
**Szabolcska u. 7**
**H-1114 Budapest (HU)**
Erfinder : **Törley, Jòzsef, Dipl.-Ing.**
**Katona J. u. 41**
**H-1137 Budapest (HU)**
Erfinder : **Fekete, György, Dr.**
**Széher u. 62**
**H-1021 Budapest (HU)**
Erfinder : **Szporny, Lászlò, Dr.**
**Szabolcska u. 7**
**H-1114 Budapest (HU)**
Erfinder : **Vereczkey, Lászlò, Dr.**
**Lajos u. 109**
**H-1036 Budapest (HU)**
Erfinder : **Pálosi, Eva, Dr.**
**Vend u. 21**
**H-1025 Budapest (HU)**
Erfinder : **Klebovich, Imre, Dr.**
**Karvaly u. 4**
**H-1125 Budapest (HU)**
Erfinder : **Vittay, Pál, Dr.**
**Jòzsef krt. 14**
**H-1085 Budapest (HU)**
Erfinder : **Görög, Sándor, Dr. Dipl.-Chem.**
**Vajda P. u. 43**
**H-1089 Budapest (HU)**
Erfinder : **Hajdu, István, Dipl.-Chem.**
**Tátra tér B/4**
**H-1205 Budapest (HU)**

(74) Vertreter : **Beszédes, Stephan G. Dr.**
**Münchener Strasse 80a Postfach 1168**
**D-8060 Dachau (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft neue 4-Hydroxy-α-äthyl-benzhydrole, ein Verfahren zu ihrer Herstellung und diese Verbindungen enthaltende Arzneimittel.

Gegenstand der Erfindung sind 4-Hydroxy-α-äthyl-benzhydrole der allgemeinen Formel

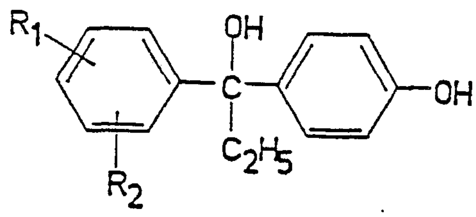

(I)

worin $R_1$ und $R_2$, die gleich oder verschieden sind und für Wasserstoffatome, Halogenatome, Trihalogenmethylreste, Alkyl- oder Alkoxyreste mit je 1 bis 4 Kohlenstoffatom(en) stehen, mit den Maßgaben, daß,

a) falls $R_1$ für ein Wasserstoffatom steht, $R_2$ von einem Wasserstoffatom oder einem Trifluormethylrest in der 3-Stellung verschieden ist, und,

b) falls $R_1$ für einen Methylrest in der 2-Stellung steht, $R_2$ von einem Methylrest in der 5-Stellung verschieden ist.

Die Herstellung von Verbindungen ähnlicher Struktur ist in folgenden Literaturstellen beschrieben : C.A. 22, 410[1] ; 35, 1781[2] ; 40, 4712[5] ; 42, P 1015 b ; 47, 9548 e ; 50, 12390 c ; 50, 2509 i ; 55, 17915 e ; 55, 15413 b ; 75, P 103682 b ; 76, P 119921 k ; 82, 16477 q ; 90, 86062 q ; 92, 52927 b. Nirgends wird jedoch eine pharmakologische Wirkung der hergestellten Verbindungen erwähnt.

Vorzugsweise ist beziehungsweise sind das beziehungsweise die Halogenatom(e), für das beziehungsweise die $R_1$ und/oder $R_2$ stehen kann beziehungsweise können, [ein] Chlor-, Fluor- und/oder Bromatom(e), insbesondere [ein] Chlor- und/oder Fluoratom(e).

Es ist auch bevorzugt, daß der beziehungsweise die Trihalogenmethylrest(e), für den beziehungsweise die $R_1$ und/oder $R_2$ stehen kann beziehungsweise können, [ein] Trifluormethylrest(e) und/oder Trichlormethylrest(e), insbesondere der beziehungsweise die erstere(n), ist beziehungsweise sind.

Ferner ist es bevorzugt, daß der beziehungsweise die Alkyl- und/oder Alkoxyrest(e), für den beziehungsweise die $R_1$ und/oder $R_2$ stehen kann beziehungsweise können, ein solcher beziehungsweise solche mit 1 oder 2, insbesondere 1, Kohlenstoffatom(en) ist beziehungsweise sind.

Besonders bevorzugte erfindungsgemäße 4-Hydroxy-α-äthyl-benzhydrole sind

3-Chlor-4'-hydroxy-α-äthyl-benzhydrol,
4-Fluor-4'-hydroxy-α-äthyl-benzhydrol,
4-Chlor-4'-hydroxy-α-äthyl-benzhydrol,
4-Brom-4'-hydroxy-α-äthyl-benzhydrol,
4-Trifluormethyl-4'-hydroxy-α-äthyl-benzhydrol,
2-Methoxy-4'-hydroxy-α-äthyl-benzhydrol und
2-Trifluormethyl-4'-hydroxy-α-äthyl-benzhydrol.

Gegenstand der Erfindung ist auch ein Verfahren zur Herstellung der erfindungsgemäßen Verbindungen, welches dadurch gekennzeichnet ist, daß in an sich bekannter Weise

a) 4-Hydroxypropiophenon mit eine Phenylgruppe aufweisenden metallorganischen Verbindungen der allgemeinen Formel

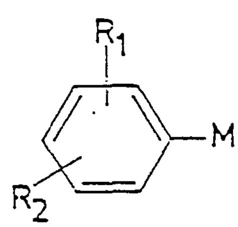

(II)

worin

$R_1$ und $R_2$ wie oben festgelegt sind und

M für Alkalimetallatom, vorzugsweise Lithium-, Natrium- oder Kaliumatom, oder einen Rest der Formel MgX mit X = Halogenatom steht,
umgesetzt wird oder

b) 4-Hydroxybenzophenone der allgemeinen Formel

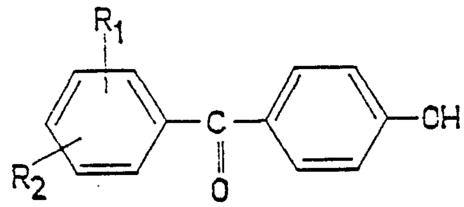

(III)

worin $R_1$ und $R_2$ wie oben festgelegt sind, mit eine Äthylgruppe aufweisenden metallorganischen Verbindungen, insbesondere Äthylmagnesiumhalogeniden oder Äthyllithium, umgesetzt werden oder

c) α-[Äthyl]-4-[benzyloxy]-benzhydrole der allgemeinen Formel

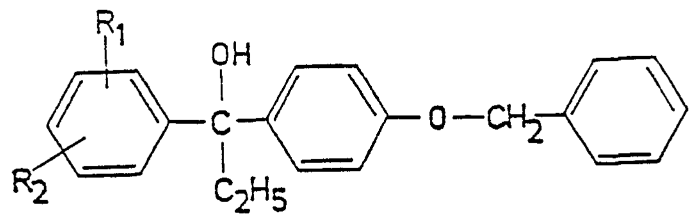

(IV)

worin $R_1$ und $R_2$ wie oben festgelegt sind, reduziert werden.

In « Chemical Abstracts », Band 96, Nr. 5, 1. Februar 1982, Seite 16, Nr. 28204a wird über den Stoffwechsel von 4-(2'-Diethylamino)-ethoxy-α-ethylbenzhydrol in Rattenversuchen berichtet. Die in dieser Literaturstelle beschriebenen Benzhydrole unterscheiden sich von den erfindungsgemäßen hauptsächlich durch das Vorliegen von Diethylaminoresten. Außerdem enthält diese Literaturstelle keinen Hinweis auf die pharmakologische Wirkung als Mittel zur Behandlung akuter Ethanolintoxikationen.

Die DE-A 2 216 138 beschreibt Bisphenolalkane der dort angegebenen Formeln I und II, die sich von den erfindungsgemäßen Benzhydrolen dadurch unterscheiden, daß in letzteren nur in der α-Stellung eine Hydroxygruppe vorliegt und sie im Gegensatz zu bekannten Verbindungen der Formel I keine OH-Gruppe an dem einen der in der α-Stellung sitzenden Benzolringe und im Gegensatz zu den bekannten Verbindungen der Formel II eine Hydroxygruppe in der 4-Stellung eines der Benzolringe aufweisen. Darüber hinaus wird für diese Bisphenolalkane eine andere Indikation angegeben, und zwar zur Behandlung und Prophylaxe von menschlichen und tierischen arteriosklerotischen Erkrankungen.

Die Ausgangsverbindungen sind bekannt beziehungsweise können nach literaturbekannten Verfahren hergestellt werden. Die Verbindungen der allgemeinen Formel (II) erhält man zum Beispiel, indem man aus den entsprechend substituierten Arylhalogeniden in an sich bekannter Weise das Grignard-Reagens bildet (M.S. Kharash et al. : Grignard reactions of nonmetallic substances, Ed. Prentice-Hall. Inc., 1954, S. 5-90). Die ein Alkalimetall enthaltenden metallorganischen Verbindungen können zum Beispiel nach Houben-Weyl : Methoden der organischen Chemie, Bd. XIII/1, S. 134-159 sowie 389-405 (1970) hergestellt werden.

Die Hydroxyketone der allgemeinen Formel (III) sind zum Beispiel durch die Fries-Reaktion synthetisierbar (A.H. Blatt : The Fries reaction in organic reactions, Vol. I, p. 342). Die Ausgangsverbindungen der allgemeinen Formel (IV) sind zum Beispiel durch die Umsetzung von 4-Benzyloxypropiophenon mit einem entsprechend substituierten Phenylmagnesiumhalogenid zugänglich ; dies ist in dem oben zitierten Buch von M.S. Kharash et al. auf den Seiten 138-143 beschrieben.

Die Verfahrensvariante a) führt man bevorzugt aus, indem man das 4-Hydroxypropiophenon mit wenigstens dem Doppelten der äquimolaren Menge der metallorganischen Verbindung der allgemeinen Formel (II) in einem wasserfreien inerten organischen Lösungsmittel, zweckmäßig unter Schutzgas, umsetzt. Als metallorganische Verbindung setzt man zweckmäßig das entsprechend substituierte Phenyllithium oder die entsprechend substituierten Phenylmagnesiumhalogenide, insbesondere das Chlorid oder Bromid, ein. Die Umsetzung wird in einem aprotischen organischen Lösungsmittel, zum Beispiel Hexamethylphosphoramid, Dimethylsulfoxyd, aliphatischen oder cycloaliphatischen Äthern, wie Diäthyläther, Dibutyläther, Äthylenglykoldimethyläther, Dioxan oder Tetrahydrofuran, in aliphatischen oder aromatischen Kohlenwasserstoffen, zum Beispiel Ligroin, Benzol, Toluol, Xylol oder in Gemischen der aufgeführten Lösungsmittel, vorgenommen. Als Schutzgas wird zum Beispiel Stickstoff oder Argon verwendet. Die Reaktionstemperatur liegt zwischen —70 °C und dem Siedepunkt des Lösungsmittels

und beträgt vorzugsweise —40 bis 100 °C. Das Reaktionsgemisch wird in an sich bekannter Weise aufgearbeitet, zum Beispiel, indem man die metallorganische Verbindung durch Eingießen des Reaktionsgemisches in verdünnte organische oder anorganische Säuren, wie Schwefelsäure, Salzsäure oder Essigsäure, oder vorzugsweise in die wäßrige Lösung von Ammoniumchlorid zersetzt und die gebildete Verbindung isoliert. Das Produkt kann durch Chromatographie oder durch Umkristallisieren gereinigt werden.

Bei der Durchführung der Verfahrensvariante b) geht man zweckmäßig so vor, daß man das Benzophenon der allgemeinen Formel (III) mit wenigstens dem Doppelten der äquimolaren Menge des Äthylmagnesiumhalogenides oder Äthyllithiums umsetzt. Die Reaktion erfolgt in einem inerten organischen Lösungsmittel, zweckmäßig unter Schutzgasatmosphäre. Als Äthylmagnesiumhalogenid setzt man zweckmäßig das Jodid oder das Bromid ein. Geeignete Lösungsmittel und Reaktionstemperaturen und inerte Gase sind die unter a) genannten. Das Reaktionsgemisch wird auf die bereits beschriebene Weise aufgearbeitet.

Im Sinne der Verfahrensvariante c) werden die Verbindungen der allgemeinen Formel (IV) reduziert. Das reduktive Abspalten der Benzylgruppe wird vorzugsweise durch katalytisches Hydrieren vorgenommen. Als Hydrierkatalysator kommen Metalle, zum Beispiel Ruthenium, Palladium, Platin, Nickel, Eisen, Kupfer, Kobalt, Chrom, Zink, Molybdän und Wolfram beziehungsweise deren Oxyde und Sulfide in Betracht. Der einzusetzende Katalysator kann zum Beispiel bereitet werden, indem man die stabilen Oxyde unmittelbar im Reaktionsgefäß mit Wasserstoff reduziert. Auf diese Weise geht man dann vor, wenn als Katalysator feinverteiltes Platin oder Palladium verwendet werden soll. Die katalytische Hydrierung kann auch in Gegenwart von Katalysatoren vorgenommen werden, die vorher auf die Oberfläche eines Trägers ausgefällt wurden. Als Träger kommen zum Beispiel Tierkohle, Siliciumdioxyd, Aluminiumoxyd sowie die Sulfate und Carbonate der Erdalkalimetalle in Frage. Die Reduktion kann auch in Gegenwart von Raney-Nickel vorgenommen werden. Vorzugsweise reduziert man in Gegenwart von Palladium, zweckmäßig Palladiumkohle, oder Raney-Nickel in einem hinsichtlich der Reaktion inerten organischen Lösungsmittel. Als solches sind zum Beispiel die niederen aliphatischen Alkohole, Äther oder Ester, ferner aliphatische, cycloaliphatische oder aromatische Kohlenwasserstoffe sowie Gemische der aufgeführten Lösungsmittel geeignet. Man hydriert unter atmosphärischem oder unter erhöhtem Druck, vorzugsweise unterhalb 506,6 kPa, bei Temperaturen zwischen 20 °C und dem Siedepunkt des Lösungsmittels. Vorzugsweise hydriert man bei Raumtemperatur unter atmosphärischem Druck bis zur Beendigung der Wasserstoffaufnahme. Dann wird der Katalysator abfiltriert und das Filtrat eingedampft. Das Produkt kann zum Beispiel durch Destillation oder Umkristallisieren gereinigt werden.

Die Verbindungen der allgemeinen Formel (I) sind zur Behandlung akuter Äthanolintoxikation geeignet und für diesen Zweck ausgedehnt verwendbar. Die akute Alkoholintoxikation ist vor allem durch Euphorie, allgemeine Stimuliertheit, Ataxie, Somnolenz und paralytische Zustände charakterisiert. Die Gefahren dieses toxischen, krankhaften Zustandes sind bekannt und nicht zu vernachlässigen. Die intoxierte Person gefährdet ihre Umgebung (z. B. Trunkenheit am Steuer) und sich selbst.

Die akute Alkoholintoxikation ist für den ischämischen Gehirninfarkt ein bedeutender Risikofaktor (Hillbom, M. u. a. : Lancet 2, 1181/1978/ ; Stroke 12, 422/1981/). Für den alkoholintoxierten Zustand gibt es kein geeignetes Antidotum. Die durch Alkohol ausgelöste lokomotorische Hyperaktivität wird an Mäusen durch α-Methyl-para-thyrosin in einem Dosisbersich normalisiert, in dem die Verbindung die spontane lokomotorische Aktivität der Tiere beeinträchtigt (Carlsson, A. u. a. : Psychopharm. 26, 307/1972/). Die narkotisierende Wirkung des Alkohols wird durch Stimulantien (Coffein, Amphetamin) verringert, die motorische Inkoordination (Ataxie) jedoch gleichzeitig prolongiert (Wallagsen, H. u. a. : Actions of alcohol, Amsterdam, Elsevier, 1970 ; Rech. R.H. u. a. : Ann. N.Y. Acad. Sci. 28, 426, 1976 ; Todzy, I. u. a. : Psychopharm. 59, 143, 1978). Die alkoholische Intoxikation, die Narkose wird durch L-Cystein verkürzt (Sprince, H. u. a. : Agents and Actions 4, 125, 1974 ; Nagasawa, H.T. u. a. : Life Sci. 17, 707, 1975) ; L-Cystein wurde zu den folgenden Untersuchungen über Schlaf unter Alkoholeinfluß als Referenzsubstanz verwendet.

Zur Messung der Änderung der durch Alkohol hervorgerufenen Narkosedauer wurden 16 Stunden lang ausgehungerte, 160-180 g schwere Hann.-Wistar-Ratten beiderlei Geschlechts verwendet. Jede Gruppe bestand aus 10 Tieren, die mit entsprechenden Dosen der Versuchsverbindungen oral behandelt wurden. Eine Stunde nach der Behandlung wurde den Tieren in einer Dosis von 3,5 g/kg intraperitoneal Äthanol appliziert. Die Schlafdauer wurde vom Ausfall des Aufrichtreflexes (righting Reflexes) bis zur spontanen Korrektur der Körperhaltung gemessen. Für jede Gruppe wurden die durchschnittliche Schlafzeit, die Standardabweichung und das Ergebnis in Prozent der Kontrolle berechnet. Die Kontrolle erhielt Placebo und ebenfalls 3,5 g/kg Alkohol. Die Ergebnisse sind in der folgenden Tabelle angegeben, in der $\bar{x} \pm$ S.E. Durchschnittswert ± Standardabweichung bedeutet.

Schlafzeit der Kontrolle : 92,4 ± 4,83 ($\bar{x} \pm$ S.E.) min.

A = 2-Trifluormethyl-4'-hydroxy-α-äthyl-benzhydrol
B = 3-Chlor-4'-hydroxy-α-äthyl-benzhydrol

(Siehe Tabelle Seite 5 f.)

Tabelle

| Behandlung | Dosis mg/kg | Schlafdauer in Prozent der Kontrolle ($\bar{x} \pm$ S.E.) |
|---|---|---|
| A | 5,0 | 69 ± 4,5 |
| | 20,0 | 52 ± 6,0 |
| | 40,0 | 39 ± 3,4 |
| B | 40,0 | 60 ± 7,5 |
| L-Cystein | 500,0 | 63 ± 5,9 |
| | 1000,0 | 66 ± 5,9 |
| Kontrolle | | 100 ± 5,2 |

Aus den Daten ist ersichtlich, daß die Verbindungen der allgemeinen Formel (I) die Zeitdauer der durch Äthanol ausgelösten Narkose wesentlich verkürzen und ihre Wirkung im Gegensatz zu der des L-Cysteins dosisabhängig ist. Die Verbindungen der allgemeinen Formel (I) erreichen beziehungsweise übertreffen die Wirkung des L-Cysteins schon in viel geringerer Dosis.

Die akute Toxizität der Verbindungen der allgemeinen Formel (I) wurde an 160-180 g schweren Wistar-Ratten beiderlei Geschlechts untersucht. Jede Gruppe bestand aus 10 Tieren, die mit je 500 mg/kg der Testverbindung in einmaliger Dosis oral behandelt wurden. Die Tiere wurden 14 Tage lang beobachtet. Von den mit der Verbindung A beziehungsweise B behandelten Tieren verendete kein einziges. Daraus ist ersichtlich, daß die Toxizität der erfindungsgemäßen Verbindungen, bezogen auf die wirksame Dosis, sehr günstig ist, ihr therapeutischer Index ist gut.

Durch Untersuchungen auf die im folgenden angegebene Weise wurde festgestellt, daß die Verbindungen auch in einer Dosis von 160 mg/kg keinerlei sonstige Wirkungen auf das Zentralnervensystem ausüben : Elektroschock (Swinyard, E.A., Brown, W.C., Goodman, L.S. : J. Pharmacol. Exp. Ther. 106, 319/1952/), Metrazolkrampf (Everett, G.M., Richards, R.K. : J. Pharmacol. Exp. Ther. 81. 402/1944/), Thiosemicarbazidkrampf (Da Vanzo, J.P., Greiz, M.E., Cormin, M.A. : Amer. J. Physiol. 201, 833/1961/), Strychninkrampf (Kerley, T.L., Richards, A.G., Begley, R.W., Abreu, B.B., Wesver, L.C. : J. Pharmacol. Exp. Ther. 132, 360/1961/), Nikotinkrampf (Stone, C.A., Mecklenburg, K.L., Torhans, M.L. : Arch. Int. Pharmacodyn. 117, 419/1958/), Drehstab (Kinnard, W.J., Carr, C.J. : J. Pharmacol. Exp. Ther. 121, 354/1957/), Physostigminletalität (Nose, T. und Kojima, M. : Europ. J. Pharmacol. 10, 83/1970/), yohimbinpotenzierende Wirkung (Quinton, R.M. : Brit. J. Pharmacol. 21, 51/1963/), schmerzstillende Wirkung (Bianchi, C., Franceschini, J. : Brit. J. Pharm. Chemother. 9, 280/1954/).

Die erfindungsgemäßen Verbindungen können zu Arzneimittelpräparaten zubereitet werden. Die Präparate können oral, rektal und/oder parenteral verabreicht werden. Zur oralen Darreichung können Tabletten, Dragees oder Kapseln hergestellt werden. Bei der Herstellung von oral verabreichbaren Formen werden als Streckmittel zum Beispiel Milchzucker oder Stärke verwendet. Als Binde- oder Granuliermittel kommen zum Beispiel Gelatine, Carboxymethylcellulose-natrium, Methylcellulose, Polyvinylpyrrolidon oder Stärkekleister in Betracht. Als Sprengmittel werden in erster Linie Kartoffelstärke oder mikrokristalline Cellulose zugesetzt, jedoch können beispielsweise auch Ultraamylopektin oder Formaldehydcasein verwendet werden. Als Antihaftmittel und Gleitmittel kommen z. B. Talk, kolloidale Kieselsäure, Stearin, Ca- und Mg-Stearat in Frage.

Die Tabletten können zum Beispiel durch Naßgranulieren und anschließendes Pressen hergestellt werden. Das Gemisch aus Wirkstoff und Füllstoffen sowie gegebenenfalls ein Teil des Sprengmittels werden mit der wäßrigen, alkoholischen oder wäßrig-alkoholischen Lösung der Bindemittel in einer geeigneten Vorrichtung granuliert, und das Granulat wird getrocknet. Zu dem trockenen Granulat werden der Rest des Sprengmittels, ferner die Antihaftmittel und das Gleitmittel gegeben, und das Gemisch wird zu Tabletten gepreßt. Gegebenenfalls können die Tabletten zur Erleichterung der Dosierung mit Teilungsmarkierungen versehen werden. Die Tabletten können auch unmittelbar durch Pressen eines Gemisches aus dem Wirkstoff und geeigneten Hilfstoffen hergestellt werden.

Die Tabletten können gewünschtenfalls unter Verwendung der in der Arzneimittelherstellung üblichen Schutz-, Geschmacks- und Farbstoffe, zum Beispiel Zucker, Cellulosederivate, wie Methyl- oder Äthylcellulose oder Carboxymethylcellulosenatrium, Polyvinylpyrrolidon, Calciumphosphat, Calciumcarbonat, Lebensmittelfarbstoffe, Lebensmittelfarblacke, Aromastoffe und Eisenoxydpigmente, auf die übliche Weise dragiert werden.

Zur Herstellung von Kapseln wird das Gemisch aus Wirkstoff und Hilfsstoffen in Kapseln gefüllt.

Zur rektalen Anwendung werden Suppositorien hergestellt. Diese enthalten außer dem Wirkstoff eine Trägermasse, das sog. Adeps pro suppositori. Als dieses kommen Pflanzenfette, zum Beispiel gehärtete Pflanzenöle, die Triglyceride von Fettsäuren mit 12-18 Kohlenstoffatomen, vorzugsweise die Trägerstoffe der Markenbezeichnung Witepsol®, in Betracht. Der Wirkstoff wird in der geschmolzenen Trägermasse

homogen verteilt und das erhaltene Gemisch zu Suppositorien vergossen.

Zur parenteralen Verabreichung werden Präparate in Injektionslösungsform hergestellt. Dazu werden die Wirkstoffe, gegebenenfalls in Gegenwart von Lösungsvermittlern, wie Polyoxyäthylensorbitanmonolaurat, -monooleat oder -monostearat (Tween 20®, Tween 60®, Tween 80®), in destilliertem Wasser und/oder unterschiedlichen organischen Lösungsmitteln, zum Beispiel Glykoläthern, gelöst. Die Injektionslösungen können ferner unterschiedliche Hilfsstoffe, zum Beispiel Konservierungsmittel, wie Benzylalkohol, p-Oxybenzoesäuremethyl- oder -propylester, Benzalkoniumchlorid oder Phenylmercuriborat, Antioxydantien, wie Ascorbinsäure, Tocopherol oder Natriumpyrosulfat, zum Binden von Metallspuren Komplexbildner, z. B. Äthylendiamintetraessigsäure, Puffersubstanzen oder Stoffe zum Einstellen des pH-Wertes sowie gegebenenfalls Lokalanästhetika, wie Lidocain, enthalten. Die Injektionslösungen werden vor dem Abfüllen filtriert und, nachdem sie in Ampullen gefüllt wurden, sterilisiert.

Die Tagesdosis beträgt, abhängend vom Zustand des Kranken, 0,1-300 mg/kg, vorzugsweise 2,0-160 mg/kg, und wird zweckmäßig in kleineren Einzeldosen verabreicht.

Die Erfindung wird an Hand der folgenden Beispiele näher erläutert.

### Beispiel 1

3-Chlor-4′-hydroxy-α-äthyl-benzhydrol

Zu dem aus 14,6 g Magnesiumspänen und 115 g 3-Chlorbrombenzol in 350 ml wasserfreiem Tetrahydrofuran bereiteten Grignardreagens gibt man unter schwachem Sieden am Rückfluß tropfenweise die Lösung von 30,1 g 4-Hydroxypropiophenen in 540 ml wasserfreiem Tetrahydrofuran. Das Reaktionsgemisch wird 30 Minuten lang eben am Sieden erhalten, dann abgekühlt und in ein Gemisch aus Eis und Eisessig gegossen. Die Phasen werden voneinander getrennt, und die organische Phase wird mit gesättigter Kochsalzlösung gewaschen, über wasserfreiem Magnesiumsulfat getrocknet und das Lösungsmittel unter vermindertem Druck abdestilliert. Der feste Rückstand wird aus einem Gemisch von n-Hexan und Äthylacetat kristallisiert. Man erhält 42,6 g der Titelverbindung, die bei 132-134 °C schmilzt.

Elementaranalyse für $C_{15}H_{15}ClO_2$

berechnet : C 68,57 H 5,75 Cl 13,49 %

gefunden : C 68,66 H 5,57 Cl 13,71 %.

### Beispiel 2

4-Fluor-4′-hydroxy-α-äthyl-benzhydrol

Zu der aus 2,8 g metallischem Lithium, 21,8 g Äthylbromid und 265 ml wasserfreiem Äther bereiteten Äthyllithiumlösung wird unter ständigem Rühren bei einer Temperatur zwischen —30 und —40 °C in einer Schutzgasatmosphäre (Argon) tropfenweise die Lösung von 10,8 g 4-Fluor-4′-hydroxybenzophenon in 50 ml wasserfreiem Tetrahydrofuran gegeben. Nach Beendigung der Zugabe läßt man die Temperatur auf 0 °C ansteigen und rührt das Gemisch bei dieser Temperatur 30 Minuten lang. Dann wird die metallorganische Verbindung unter Kühlung mit 20 %iger wäßriger Ammoniumchloridlösung zersetzt. Die wäßrige Phase wird mit Äther extrahiert. Die ätherischen Phasen werden vereinigt, mit Wasser neutral gewaschen, über wasserfreiem Magnesiumsulfat getrocknet und unter vermindertem Druck eingedampft. Der feste Rückstand wird aus einem Gemisch von n-Hexan und Äthylacetat kristallisiert. Man erhält 8,2 g der Titelverbindung, die bei 125-126 °C schmilzt.

Elementaranalyse für die Summenformel $C_{15}H_{15}FO_2$

berechnet : C 73,15 H 6,14 F 7,71 %

gefunden : C 73,26 H 6,18 F 7,95 %.

### Beispiel 3

4-Chlor-4′-hydroxy-α-äthyl-benzhydrol

Zu der aus 3,9 g Magnesiumspänen, 17,4 g Äthylbromid und 50 ml wasserfreiem Äther bereiteten Äthylmagnesiumbromidlösung wird unter Stickstoffatmosphäre bei —10 °C unter Rühren tropfenweise die Lösung von 9,3 g 4-Chlor-4′-hydroxybenzophenon in 40 ml wasserfreiem Äther gegeben. Das Reaktionsgemisch wird auf Raumtemperatur erwärmt und bei dieser Temperatur eine halbe Stunde lang gerührt. Das Gemisch wird in eine eiskalte wäßrige Ammoniumchloridlösung eingegossen. Die wäßrige Phase wird mit Äther extrahiert, die vereinigten ätherischen Phasen werden mit Wasser neutral gewaschen, über wasserfreiem Magnesiumsulfat getrocknet, filtriert und im Vakuum von Lösungsmittel befreit. Das Rohprodukt wird aus einem Gemisch von Benzol und Äthylacetat kristallisiert. Man erhält 5,5 g der Titelverbindung, die bei 148-149 °C schmilzt.

Elementaranalyse für die Summenformel $C_{15}H_{15}ClO_2$

berechnet : C 68,57 H 5,75 Cl 13,49 %

gefunden : C 68,78 H 5,87 Cl 13,57 %.

Beispiel 4

4-Brom-4'-hydroxy-α-äthyl-benzhydrol

Zu 400 ml einer 0,5 molaren ätherischen 4-Bromphenyllithiumlösung wird unter Rühren bei Temperaturen zwischen — 50 und — 40 °C unter Argonatmosphäre tropfenweise die Lösung von 7,5 g 4-Hydroxypropiophenon in 37 ml wasserfreiem Tetrahydrofuran gegeben. Das Reaktionsgemisch wird bei 0 °C eine Stunde lang gerührt und dann mit eiskalter Essigsäure versetzt. Die wäßrige Phase wird mit Äther extrahiert. Die ätherischen Phasen werden vereinigt, mit Wasser neutral gewaschen, über wasserfreiem Magnesiumsulfat getrocknet und unter vermindertem Druck vom Lösungsmittel befreit. Der Rückstand wird an einer Silikagelsäule mit dem im Volumverhältnis 7 : 3 bereiteten Gemisch aus Benzol und Äthylacetat chromatographiert. Dann wird das Lösungsmittel im Vakuum abdestilliert und der Rückstand aus einem Äthylacetat/Dichlormethan-Gemisch kristallisiert. Man erhält 4,2 g des Titelverbindung, die bei 161-162 °C schmilzt.

Elementaranalyse für die Summenformel $C_{15}H_{15}BrO_2$
berechnet : C 58,64 H 4,92 Br 26,01 %
gefunden : C 58,86 H 4,83 Br 26,17 %.

Beispiel 5

4-Trifluormethyl-4'-hydroxy-α-äthyl-benzhydrol

27 g 4-Trifluormethyl-4'-hydroxy-α-äthyl-benzhydrol werden in 270 ml Benzol gelöst und in Gegenwart von 13,5 g 10 %iger Palladiumaktivkohle bis zur Aufnahme der berechneten Menge Wasserstoff (etwa 80 Minuten lang) hydriert. Nach Abfiltrieren des Katalysators wird das Benzol unter vermindertem Druck abdestilliert und der Rückstand aus einem Gemisch von Äthylacetat und n-Hexan kristallisiert. Man erhält 19 g der Titelverbindung, die bei 126-127 °C schmilzt.

Elementaranalyse für die Summenformel $C_{16}H_{15}F_3O_2$
berechnet : C 64,86 H 5,10 F 19,24 %
gefunden : C 64,68 H 5,23 F 19,50 %.

Durch entsprechende Wahl der Ausgangsstoffe werden die folgenden Verbindungen in analoger Weise erhalten :

2-Methoxy-4'-hydroxy-α-äthyl-benzhydrol
Schmelzpunkt : 165-166 °C
Elementaranalyse für die Summenformel $C_{16}H_{18}O_3$
Berechnet : C 74,39 H 7,02 %
gefunden : C 74,46 H 7,11 %.

2-Trifluormethyl-4'-hydroxy-α-äthyl-benzhydrol
Schmelzpunkt : 131-132 °C
Elementaranalyse für die Summenformel $C_{16}H_{15}F_3O_2$
berechnet : C 64,86 H 5,10 F 19,28 %
gefunden : C 64,97 H 5,16 F 19,35 %.

Beispiel 6

Aus den erfindungsgemäßen Verbindungen können zum Beispiel die folgenden Arzneimittelpräparate hergestellt werden.

Tabletten

Zusammensetzung einer Tablette :

| | |
|---|---|
| Wirkstoff | 100,0 mg |
| Lactose | 184,0 mg |
| Kartoffelstärke | 80,0 mg |
| Polyvinylpyrrolidon | 8,0 mg |
| Talk | 12,0 mg |
| Magnesiumstearat | 2,0 mg |
| Aerosil® (kolloidales $SiO_2$) | 2,0 mg |
| Ultraamylopektin | 12,0 mg |

Durch Naßgranulieren und Pressen werden aus den angegebenen Stoffen Tabletten von 400 mg Gewicht hergestellt.

Wirkstoff : 2-Trifluormethyl-4'-hydroxy-α-äthyl-benzhydrol.

Dragees

Die auf die beschriebene Weise erhaltenen Tabletten werden in an sich bekannter Weise mit einem aus Zucker und Talk bestehenden Überzug versehen und dann mit einem Gemisch aus Bienenwachs und Carnaubawachs poliert. Gewicht eines Dragees : 500,0 mg.

Kapseln

Zusammensetzung der Füllung einer Kapsel

| | |
|---|---|
| Wirkstoff | 50,0 mg |
| Lactose | 100,0 mg |
| Talk | 2,0 mg |
| Kartoffelstärke | 30,0 mg |
| mikrokristalline Cellulose | 8,0 mg |

Der Wirkstoff wird gründlich mit den Hilfsstoffen vermischt, das Gemisch durch ein Sieb der Maschenweite 0,32 mm gesiebt und dann in Hartgelatinekapseln (Größe 4) gefüllt. Wirkstoff : 2-Trifluormethyl-4'-hydroxy-α-äthyl-benzhydrol

Suppositorien

Zusammensetzung eines Suppositoriums :

| | |
|---|---|
| Wirkstoff | 100,0 mg |
| Lactose | 200,0 mg |
| Suppositorienmasse (z. B. Witepsol® H) | 1 700,0 mg |

Die Grundmasse wird geschmolzen und die Schmelze auf 35 °C abgekühlt. Der Wirkstoff wird gründlich mit der Lactose vermischt und das Gemisch in einem Homogenisator mit der Schmelze homogenisiert. Das erhaltene Gemisch wird in gekühlt Suppositorienformen gegossen.

Gewicht eines Suppositoriums : 2 000 mg

Wirkstoff : 2-Trifluormethyl-4'-hydroxy-α-äthyl-benzhydrol.

Suspension

100 ml Suspension enthalten :

| | |
|---|---|
| Wirkstoff | 1,0 g |
| Natriumhydroxyd | 0,26 g |
| Citronensäure | 0,30 g |
| Nipagin® (Natriumsalz des 4-Hydroxybenzoesauremethylesters) | 0,10 g |
| Carbopol® 940 (Polyacrylsäure) | 0,30 g |
| Äthanol (96 %ig) | 1,00 g |
| Himbeeraroma | 0,60 g |
| Sorbit (70 %ige wäßrige Lösung) | 71,00 g |
| dest. Wasser zum Auffüllen auf | 100,00 ml |

Das Nipagin® und die Citronensäure werden in 20 ml destilliertem Wasser gelöst. Zu der Lösung gibt man in kleinen Portionen, unter intensivem Rühren das Carbopol® und läßt die Lösung dann 10-12 Stunden lang stehen. Anschließend werden die mit 1 ml destilliertem Wasser bereitete Lösung des Natriumhydroxyds, dann die wäßrige Lösung des Sorbits und schließlich die äthanolische Lösung des Himbeeraromas unter Rühren zugegeben. Zu der so bereiteten Trägersubstanz gibt man in kleinen Portionen den Wirkstoff und homogenisiert das Ganze mit einem Mixer. Die Suspension wird mit destilliertem Wasser auf 100 ml aufgefüllt und in einer Kolloidmühle homogenisiert.

Wirkstoff : 3-Chlor-4'-hydroxy-α-äthyl-benzhydrol.

**Patentansprüche** (für die Vertragsstaaten : BE, CH, LI, DE, FR, GB, IT, LU, NL, SE)

1. 4-Hydroxy-α-äthyl-benzhydrole der allgemeinen Formel

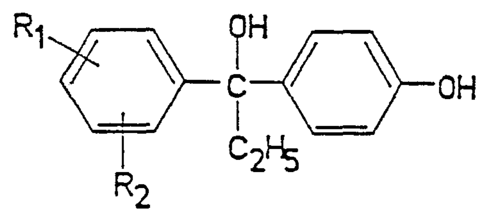

(I)

worin $R_1$ und $R_2$, die gleich oder verschieden sind und für Wasserstoffatome, Halogenatome, Trihalogenmethylreste, Alkyl- oder Alkoxyreste mit je 1 bis 4 Kohlenstoffatom(en) stehen, mit den Maßgaben, daß

a) falls $R_1$ für ein Wasserstoffatom steht, $R_2$ von einem Wasserstoffatom oder einem Trifluormethylrest in der 3-Stellung verschieden ist, und,

b) falls $R_1$ für einen Methylrest in der 2-Stellung steht, $R_2$ von einem Methylrest in der 5-Stellung verschieden ist.

2. 4-Hydroxy-α-äthyl-benzhydrole nach Anspruch 1, dadurch gekennzeichnet, daß das beziehungsweise die Halogenatom(e), für das beziehungsweise die $R_1$ und/oder $R_2$ stehen kann beziehungsweise können, [ein] Chlor-, Fluor- und/oder Bromatom(e), ist beziehungsweise sind.

3. 4-Hydroxy-α-äthyl-benzhydrole nach Anspruch 1 oder 2, dadurch gekennzeichnet, daß der beziehungsweise die Trihalogenmethylrest(e), für den beziehungsweise die $R_1$ und/oder $R_2$ stehen kann beziehungsweise können, [ein] Trifluormethylrest(e) und/oder Trichlormethylrest(e) ist beziehungsweise sind.

4. 4-Hydroxy-α-äthyl-benzhydrole nach Anspruch 1 bis 3, dadurch gekennzeichnet, daß der beziehungsweise die Alkyl- und/oder Alkoxyrest(e), für den beziehungsweise die $R_1$ und/oder $R_2$ stehen kann beziehungsweise können, ein solcher beziehungsweise solche mit 1 oder 2, insbesondere 1, Kohlenstoffatom(en) ist beziehungsweise sind.

5. Die 4-Hydroxy-α-äthyl-benzhydrole
3-Chlor-4'-hydroxy-α-äthyl-benzhydrol,
4-Fluor-4'-hydroxy-α-äthyl-benzhydrol,
4-Chlor-4'-hydroxy-α-äthyl-benzhydrol,
4-Brom-4'-hydroxy-α-äthyl-benzhydrol,
4-Trifluormethyl-4'-hydroxy-α-äthyl-benzhydrol,
2-Methoxy-4'-hydroxy-α-äthyl-benzhydrol und
2-Trifluormethyl-4'-hydroxy-α-äthyl-benzhydrol.

6. Verfahren zur Herstellung der Verbindungen nach Anspruch 1 bis 5, dadurch gekennzeichnet, daß man in an sich bekannter Weise

a) 4-Hydroxypropiophenon mit eine Phenylgruppe aufweisenden metallorganischen Verbindungen der allgemeinen Formel

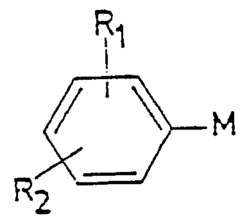

(II)

worin

$R_1$ und $R_2$ wie in den Ansprüchen 1 bis 4 festgelegt sind und

M für Alkalimetallatom, vorzugsweise Lithium-, Natrium- oder Kaliumatom, oder einen Rest der Formel MgX mit X = Halogenatom steht,

umsetzt oder

b) 4-Hydroxybenzophenon der allgemeinen Formel

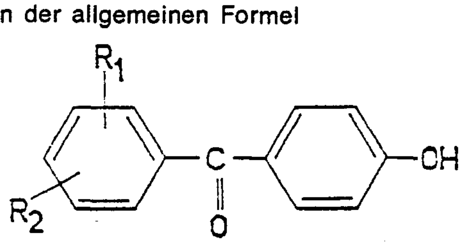

(III)

worin $R_1$ und $R_2$ wie in den Ansprüchen 1 bis 4 festgelegt sind, mit eine Äthylgruppe aufweisenden metallorganischen Verbindungen, insbesondere Äthylmagnesiumhalogeniden oder Äthyllithium, umsetzt oder

c) α-[Äthyl]-4-[benzyloxy]-benzhydrole der allgemeinen Formel

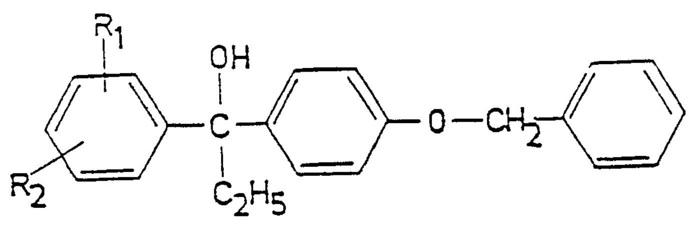

(IV)

worin $R_1$ und $R_2$ wie in den Ansprüchen 1 bis 4 festgelegt sind, reduziert.

7. Arzneimittel, gekennzeichnet durch einen Gehalt an 1 oder mehr Verbindung(en) nach Anspruch 1 bis 5 als Wirkstoff(en), zweckmäßigerweise zusammen mit 1 oder mehr üblichen pharmazeutischen Konfektionierungsmittel(n).

**Patentansprüche** (für den Vertragsstaat AT)

1. Verfahren zur Herstellung von 4-Hydroxy-α-ethyl-benzhydrolen der allgemeinen Formel

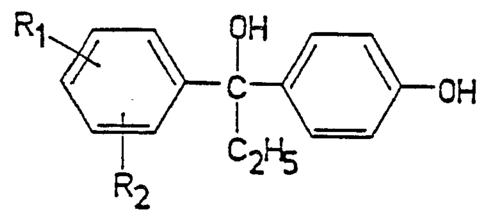

(I)

worin $R_1$ und $R_2$, die gleich oder verschieden sind, für Wasserstoffatome, Halogenatome, Trihalogenmethylreste, Alkyl- oder Alkoxyreste mit je 1 bis 4 Kohlenstoffatom(en) stehen, mit den Maßgaben, daß,

a) falls $R_1$ für ein Wasserstoffatom steht, $R_2$ von einem Wasserstoffatom oder einem Trifluormethylrest in der 3-Stellung verschieden ist, und

b) falls $R_1$ für einen Methylrest in der 2-Stellung steht, $R_2$ von einem Methylrest in der 5-Stellung verschieden ist,

dadurch gekennzeichnet, daß man in an sich bekannter Weise

a) 4-Hydroxypropiophenon mit eine Phenylgruppe aufweisenden metallorganischen Verbindungen der allgemeinen Formel

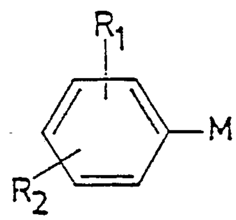

(II)

worin

$R_1$ und $R_2$ wie in den Ansprüchen 1 bis 4 festgelegt sind und

M für Alkalimetallatom, vorzugsweise Lithium-, Natrium- oder Kaliumatom, oder einen Rest der Formel MgX mit X = Halogenatom steht,

umsetzt oder

b) 4-Hydroxybenzophenone der allgemeinen Formel

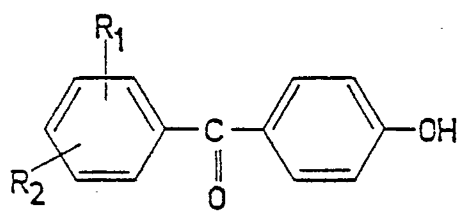

(III)

worin $R_1$ und $R_2$ wie in den Ansprüchen 1 bis 4 festgelegt sind, mit eine Äthylgruppe aufweisenden metallorganischen Verbindungen, insbesondere Äthylmagnesiumhalogeniden oder Äthyllithium, umsetzt oder

c) α-[Äthyl]-4-[benzyloxy]-benzhydrole der allgemeinen Formel

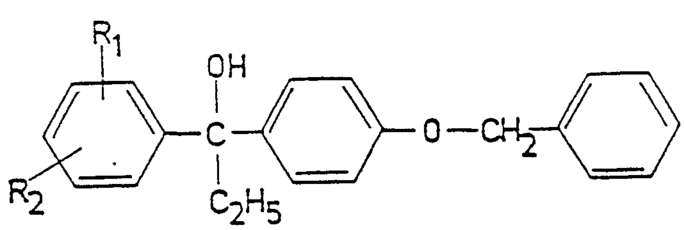

(IV)

worin $R_1$ und $R_2$ wie in den Ansprüchen 1 bis 4 festgelegt sind, reduziert.

2. Verfahren nach Anspruch 1, dadurch gekennzeichnet, daß 4-Hydroxy-α-ethyl-benzhydrole der allgemeinen Formel I hergestellt werden, in welcher das bzw. die Halogenatom(e), für das bzw. die $R_1$ und/oder $R_2$ stehen kann bzw. können, [ein] Chlor-, Fluor- und/oder Bromatom(e) ist bzw. sind.

3. Verfahren nach Anspruch 1, dadurch gekennzeichnet, daß 4-Hydroxy-α-ethyl-benzhydrole der allgemeinen Formel I hergestellt werden, in welcher der bzw. die Trihalogenmethylrest(e), für den bzw. die $R_1$ und/oder $R_2$ stehen können, [ein] Trifluormethylrest(e) und/oder Trichlormethylrest(e) ist bzw. sind.

4. Verfahren nach Anspruch 1, dadurch gekennzeichnet, daß 4-Hydroxy-α-ethyl-benzhydrole der allgemeinen Formel I hergestellt werden, in welcher der bzw. die Alkyl- und/oder Alkoxyrest(e), für den bzw. die $R_1$ und/oder $R_2$ stehen kann bzw. können, ein solcher bzw. solche mit 1 oder 2, insbesondere 1, Kohlenstoffatom(en) ist bzw. sind.

5. Verfahren nach den Ansprüchen 1 bis 4, dadurch gekennzeichnet, daß 3-Chlor-4'-hydroxy-α-ethyl-benzhydrol, 4-Fluor-4'-hydroxy-α-ethyl-benzhydrol, 4-Chlor-4'-hydroxy-α-ethyl-benzhydrol, 4-Brom-4'-hydroxy-α-ethyl-benzhydrol, 4-Trifluormethyl-4'-hydroxy-α-ethyl-benzhydrol, 2-Methoxy-4'-hydroxy-α-ethyl-benzhydrol und 2-Trifluormethyl-4'-hydroxy-α-ethyl-benzhydrol hergestellt werden.

**Claims** (for the Contracting States : BE, CH, DE, FR, GB, IT, LI, LU, NL, SE)

1. 4-Hydroxy-α-ethyl-benzhydrols of the generic formula

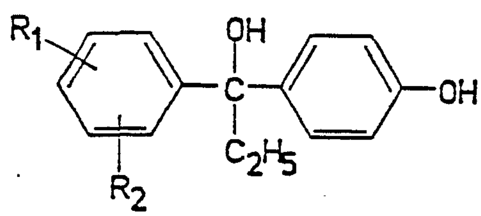

(I)

wherein $R_1$ and $R_2$, which are the same or different, are standing for hydrogen atoms, halogen atoms, trihalogenmethyl radicals, alkyl- or alkoxy radicals having each 1 to 4 carbon atom(s), with the provisos that

a) if $R_1$ is standing for a hydrogen atom, $R_2$ is different from a hydrogen atom or a trifluoromethyl radical in the 3-position, and

b) if $R_1$ is standing for a methyl radical in the 2-position, $R_2$ is different from a methyl radical in the 5-position.

2. 4-Hydroxy-α-ethyl-benzhydrols according to claim 1, characterized by the fact that the halogen atom(s) for which $R_1$ and/or $R_2$ can stand, is (are) [a] chlorine-, fluorine- and/or bromine atom(s).

3. 4-Hydroxy-α-ethyl-benzhydrols according to claim 1 or 2, characterized by the fact that the trihalogenmethyl radical(s) for which $R_1$ and/or $R_2$ can stand, is (are) [a] trifluoromethyl radical(s) and/or trichloromethyl radical(s).

4. 4-Hydroxy-α-ethyl-benzhydrols according to claim 1 to 3, characterized by the fact that the alkyl- and/or alkoxy radical(s) for which $R_1$ and/or $R_2$ can stand is a such one (are those ones) having 1 or 2, especially 1, carbon atom(s).

5. The 4-Hydroxy-α-ethyl-benzhydrols
3-chloro-4'-hydroxy-α-ethyl-benzhydrol,
4-fluoro-4'-hydroxy-α-ethyl-benzhydrol,
4-chloro-4'-hydroxy-α-ethyl-benzhydrol,
4-bromo-4'-hydroxy-α-ethyl-benzhydrol,
4-trifluoromethyl-4'-hydroxy-α-ethyl-benzhydrol,
2-methoxy-4'-hydroxy-α-ethyl-benzhydrol and
2-trifluoromethyl-4'-hydroxy-α-ethyl-benzhydrol.

6. A process for preparing the compounds according to claim 1 to 5, characterized by the fact that in a manner known per se

a) 4-hydroxypropiophenone is reacted with organometallic compounds having a phenyl group of the generic formula

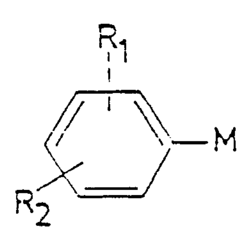

(II)

wherein

$R_1$ and $R_2$ are as defined in the claims 1 to 4 and

M is standing for an alkaline metal atom, preferably lithium-, sodium- or potassium atom, or a radical of the formula MgX, X being halogen, or

b) 4-hydroxybenzophenone of the generic formula

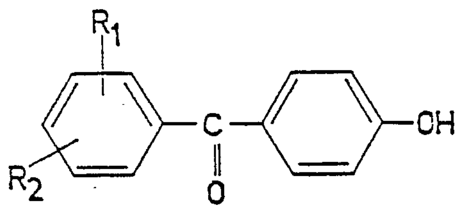

(III)

wherein $R_1$ and $R_2$ are as defined in the claims 1 to 4, are reacted with organometallic compounds having an ethyl group, especially ethylmagnesiumhalides or ethyllithium, or

c) α-[ethyl]-4-[benzyloxy]-benzhydrols of the generic formula

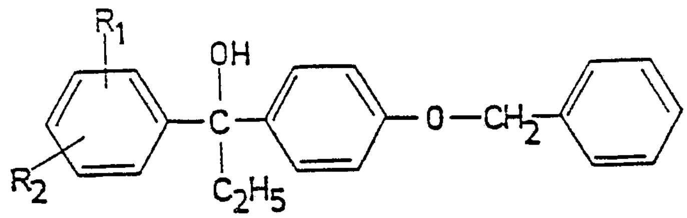

(IV)

wherein $R_1$ and $R_2$ are as defined in the claims 1 to 4, are reduced.

7. Pharmaceutical composition, characterized by a content or 1 or more compound(s) according to claim 1 to 5 as active ingredient(s), preferably together with 1 or more conventional pharmaceutical processing agent(s).

**Claims** (for the Contracting State AT)

1. A process for preparing 4-hydroxy-α-ethyl-benzhydrols of the generic formula

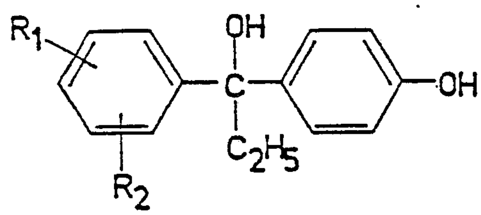

(I)

wherein $R_1$ and $R_2$, which are the same or different, are standing for hydrogen atoms, halogen atoms, trihalogenmethyl radicals, alkyl- or alkoxy radicals having each 1 to 4 carbon atom(s), with the provisos that

a) if $R_1$ is standing for a hydrogen atom, $R_2$ is different from a hydrogen atom or a trifluoromethyl radical in the 3-position, and

b) if $R_1$ is standing for a methyl radical in the 2-position, $R_2$ is different from a methyl radical in the 5-position

characterized by the fact that in a manner known per se

a) 4-hydroxypropiophenone is reacted with organometallic compounds having a phenyl group of the generic formula

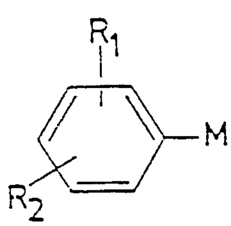

(II)

wherein

$R_1$ and $R_2$ are as defined in the claims 1 to 4 and

M is standing for an alkaline metal atom, preferably lithium-, sodium- or potassium atom, or a radical of the formula MgX, X being halogen, or

b) 4-hydroxybenzophenone of the generic formula

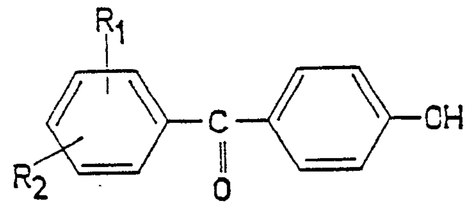

(III)

wherein $R_1$ and $R_2$ are as defined in the claims 1 to 4, are reacted with organometallic compounds having an ethyl group, especially ethylmagnesiumhalides or ethyllithium, or

c) α-[ethyl]-4-[benzyloxy]-benzhydrols of the generic formula

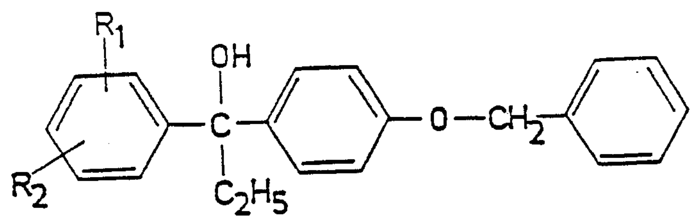

(IV)

wherein $R_1$ and $R_2$ are as defined in the claims 1 to 4, are reduced.

2. A process according to claim 1, characterized by the fact, that 4-hydroxy-α-ethyl-benzhydrols of the generic formula I are prepared in which the halogen atom(s) for which $R_1$ and/or $R_2$ can stand, is (are) [a] chlorine-, fluorine- and/or bromine atom(s).

3. A process according to claim 1, characterized by the fact that 4-hydroxy-α-ethyl-benzhydrols of the generic formula I are prepared in which the trihalogenmethyl radical(s) for which $R_1$ and/or $R_2$ can stand is (are) [a] trifluoromethyl radical(s) and/or trichloromethyl radical(s).

4. A process according to claim 1, characterized by the fact that 4-hydroxy-α-ethyl-benzhydrols of the generic formula I are prepared in which the alkyl- and/or alkoxy radical(s) for which $R_1$ and/or $R_2$ can stand is a such one (are those ones) having 1 or 2, especially 1, carbon atom(s).

5. A process according to the claims 1 to 4, characterized by the fact that 3-chloro-4'-hydroxy-α-ethyl-benzhydrol, 4-fluoro-4'-hydroxy-α-ethyl-benzhydrol, 4-chloro-4'-hydroxy-α-ethyl-benzhydrol, 4-bromo-4'-hydroxy-α-ethyl-benzhydrol, 4-trifluoromethyl-4'-hydroxy-α-ethyl-benzhydrol, 2-methoxy-4'-hydroxy-α-ethyl-benzhydrol and 2-trifluoromethyl-4'-hydroxy-α-ethyl-benzhydrol are prepared.

**Revendications** (pour les Etats contractants : BE, CH, DE, FR, GB, IT, LI, LU, NL, SE)

1. 4-Hydroxy-α-éthyl-benzhydrols de formule générale

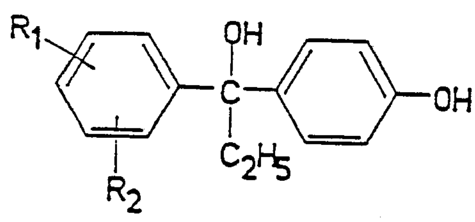

(I)

dans laquelle $R_1$ et $R_2$, qui sont identiques ou différents, représentent des atomes d'hydrogène, des atomes d'halogènes, des radicaux trihalogénométhyle, des radicaux alkyle ou alcoxy ayant chacun 1 à 4 atomes de carbone, à condition que,

a) dans le cas où $R_1$ représente un atome d'hydrogène, $R_2$ soit différent d'un atome d'hydrogène ou d'un radical trifluorométhyle en position 3, et,

b) dans le cas où $R_1$ représente un radical méthyle en position 2, $R_2$ soit différent d'un radical méthyle en position 5.

2. 4-Hydroxy-α-éthyl-benzhydrols selon la revendication 1, caractérisés en ce que le ou les atomes d'halogènes, que peuvent représenter respectivement le ou les substituants $R_1$ et/ou $R_2$, est ou sont un ou des atomes de chlore, fluor et/ou brome.

3. 4-Hydroxy-α-éthyl-benzhydrols selon la revendication 1 ou 2, caractérisés en ce que le ou les radicaux trihalogénométhyle, que peuvent représenter respectivement le ou les substituants $R_1$ et/ou $R_2$, est ou sont un ou des radicaux trifluorométhyle et/ou trichlorométhyle.

4. 4-Hydroxy-α-éthyl-benzhydrols selon l'une des revendications 1 à 3, caractérisés en ce que le ou les radicaux alkyle et/ou alcoxy, que peuvent représenter respectivement le ou les substituants $R_1$ et/ou $R_2$, est ou sont un tel radical ou de tels radicaux ayant 1 ou 2 atomes de carbone, en particulier 1 atome de carbone.

5. Les 4-hydroxy-α-éthyl-benzhydrols :
3-Chloro-4'-hydroxy-α-éthyl-benzhydrol,
4-Fluoro-4'-hydroxy-α-éthyl-benzhydrol,
4-Chloro-4'-hydroxy-α-éthyl-benzhydrol,
4-Bromo-4'-hydroxy-α-éthyl-benzhydrol,
4-Trifluorométhyl-4'-hydroxy-α-éthyl-benzhydrol,
2-Méthoxy-4'-hydroxy-α-éthyl-benzhydrol et
2-Trifluorométhyl-4'-hydroxy-α-éthyl-benzhydrol.

6. Procédé de préparation des composés selon les revendications 1 à 5, caractérisé en ce que, d'une manière connue en soi,

a) on fait réagir la 4-hydroxypropiophénone avec des composés organo-métallique, présentant un groupe phényle, de formule générale

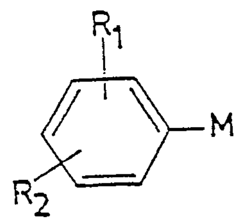

(II)

dans laquelle

$R_1$ et $R_2$ sont définis comme spécifié dans les revendications 1 à 4 et

M représente un atome d'un métal alcalin, de préférence un atome de lithium, de sodium ou de potassium, ou un radical de formule MgX dans laquelle X = un atome d'halogène, ou

b) on fait réagir des 4-hydroxybenzophénones de formule générale

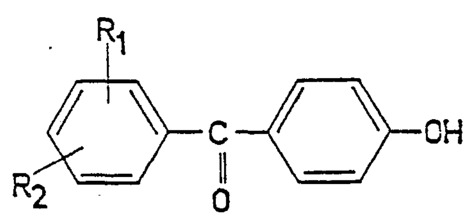

(III)

dans laquelle $R_1$ et $R_2$ sont définis comme spécifié dans les revendications 1 à 4 avec des composés organométalliques présentant un groupe éthyle, en particulier des halogénures d'éthylmagnésium ou l'éthyllithium, ou

c) on réduit des α-[éthyl]-4-[benzyloxy]-benzhydrols de formule générale

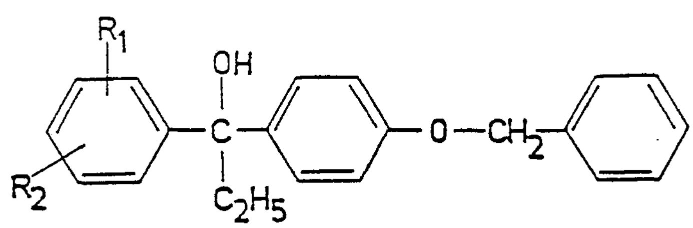

(IV)

dans laquelle $R_1$ et $R_2$ sont définis comme spécifié dans les revendications 1 à 4.

7. Médicaments, caractérisés par une teneur en un ou plusieurs composés selon les revendications 1 à 5 en tant que produits actifs, convenablement en même temps qu'un ou plusieurs agents de conditionnement pharmaceutiques courants.

**Revendications** (pour l'Etat contractant AT)

1. Procédé de préparation des 4-hydroxy-α-éthyl-benzhydrols de formule générale

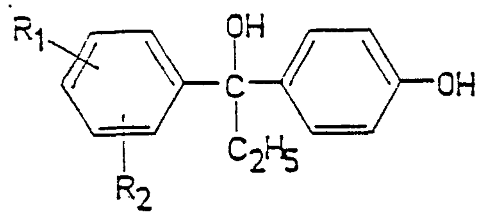

(I)

dans laquelle $R_1$ et $R_2$, qui sont identiques ou différents, représentent des atomes d'hydrogène, des atomes d'halogènes, des radicaux trihalogénométhyle, des radicaux alkyle ou alcoxy ayant chacun 1 à 4 atomes de carbone, à condition que,

a) dans le cas où $R_1$ représente un atome d'hydrogène, $R_2$ soit différent d'un atome d'hydrogène ou d'un radical trifluorométhyle en position 3, et

b) dans le cas où $R_1$ représente un radical méthyle en position 2, $R_2$ soit différent d'un radical méthyle en position 5,

caractérisé en ce que, d'une manière connue en soi,

a) on fait réagir la 4-hydroxypropiophénone avec des composés organométalliques, présentant un groupe phényle, de formule générale

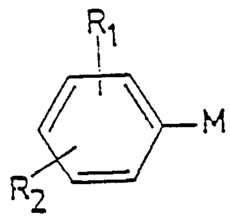

(II)

dans laquelle

$R_1$ et $R_2$ sont définis comme ci-dessus et

M représente un atome d'un métal alcalin, de préférence un atome de lithium, de sodium ou de potassium, ou un radical de formule MgX dans laquelle X = un atome d'halogène, ou

b) on fait réagir des 4-hydroxybenzophénones de formule générale

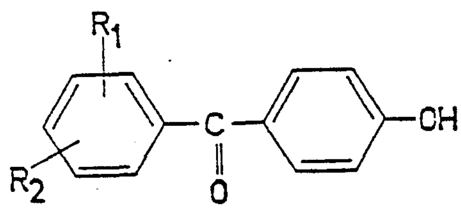

(III)

dans laquelle $R_1$ et $R_2$ sont définis comme ci-dessus avec des composés organométalliques présentant un groupe éthyle, en particulier des halogénures d'éthylmagnésium ou l'éthyllithium, ou

c) on réduit des α-[éthyl]-4-[benzyloxy]-benzhydrols de formule générale

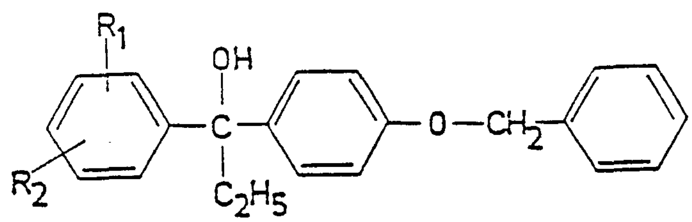

(IV)

dans laquelle $R_1$ et $R_2$ sont définis comme ci-dessus.

2. Procédé selon la revendication 1, caractérisé en ce que 4-hydroxy-α-éthyl-benzhydrols de formule générale sont préparés dans lesquels le ou les atomes d'halogènes, que peuvent représenter respectivement le ou les substituants $R_1$ et/ou $R_2$, est ou sont un ou des atomes de chlore, fluor et/ou brome.

3. Procédé selon la revendication 1 ou 2, caractérisé en ce que 4-hydroxy-α-éthyl-benzhydrols sont préparés dans lesquels le ou les radicaux trihalogénométhyle, que peuvent représenter respectivement le ou les substituants $R_1$ et/ou $R_2$, est ou sont un ou des radicaux trifluorométhyle et/ou trichlorométhyle.

4. Procédé selon l'une des revendications 1 à 3, caractérisé en ce que 4-hydroxy-α-éthyl-benzhydrols sont préparés dans lesquels le ou les radicaux alkyle et/ou alcoxy, que peuvent représenter respectivement le ou les substituants $R_1$ et/ou $R_2$, est ou sont un tel radical ou de tels radicaux ayant 1 ou 2 atomes de carbone, en particulier 1 atome de carbone.

5. Procédé selon l'une des revendications 1 à 4, caractérisés en ce que les 4-hydroxy-α-éthyl-benzhydrols : 3-Chloro-4'-hydroxy-α-éthyl-benzhydrol, 4-Fluoro-4'-hydroxy-α-éthyl-benzhydrol, 4-Chloro-4'-hydroxy-α-éthyl-benzhydrol, 4-Bromo-4'-hydroxy-α-éthyl-benzhydrol, 4-Trifluorométhyl-4'-hydroxy-α-éthyl-benzhydrol, 2-Méthoxy-4'-hydroxy-α-éthyl-benzhydrol et 2-Trifluorométhyl-4'-hydroxy-α-éthyl-benzhydrol, sont préparés.